(19) Europäisches Patentamt
European Patent Office
Office européen des brevets

(11) Publication number: **0 122 023 A1**

(12) **EUROPEAN PATENT APPLICATION**

(21) Application number: 84301507.4

(22) Date of filing: 07.03.84

(51) Int. Cl.³: C 12 Q 1/12, C 12 Q 1/36, C 12 Q 1/34

(30) Priority: 07.03.83 US 472663

(43) Date of publication of application: 17.10.84 Bulletin 84/42

(84) Designated Contracting States: AT BE CH DE FR GB IT LI LU NL SE

(71) Applicant: E-Y LABORATORIES, INC., 127 North Amphlett Boulevard, San Mateo California 94401 (US)

(72) Inventor: Chu, Albert En-Yuen, 140 Roblar Avenue, Hillsborough California 94010 (US)
Inventor: Chun, Peter Kwok Chi, 2566 Adams Court, South San Francisco California 94080 (US)

(74) Representative: Bizley, Richard Edward et al, BOULT, WADE & TENNANT 27 Furnival Street, London EC4A 1PQ (GB)

(54) **Microorganisms detection methods and the separation of agglutinated microorganisms, and a kit for use therein.**

(57) A test for the differential detection of suspected N. gonorrhoeae (N.g.) and N. meningitidis (N.m.) in provided by contacting a suspected sample with a lectin, preferably wheat germ agglutinin and either soy bean agglutinin or STA potato lectin. An agglutinin reaction is a positive indication of the presence of such microorganisms. Separate portions of the specimen may be contaced with fast substrates specific for (a) N.g., (b) N.m., and (c) B. catarrhalis. Suitable fast substrates are proline naphthylamide, gamma glutamyl naphthylamide, and glycyl prolyl naphthylamide, respectively, each substituted with an electron donating group at the 4-position, 6-position, or both. Another portion of the specimen may be reacted with a substrate for beta-galactosidase enzyme, a positive reaction being an indication that the specimen contains N.lactimica. Autoagglutination, which could otherwise give a false positive in the lectin test, may be prevented by pretreatment of the specimen with DNA'ase and/or an anhydride such as succinic anhydride.

EP 0 122 023 A1

# MICROORGANISM DETECTION METHODS AND THE SEPARATION OF AGGLUTINATED MICROORGANISMS, AND A KIT FOR USE THEREIN

The present invention relates to the detection of microorganisms, specifically Neisseria gonorrhoeae (N.g.) and/or Neisseria meningitidis (N.m.) microorganisms. One known method for detecting N.g. infections is by contacting the specimen with a lectin, wheat germ agglutinin (WGA), to agglutinate the organisms. In the technique disclosed in Doyle et al U.S. Patent 4,298,689, the presence of an agglutination reaction between the WGA and the specimen is stated to be a presumptive indication of the presence of N.g. microorganisms. A problem with this test has been reported in Curtis, G. D. W., et al, Br. J. Vener. Dis., 1981; 57:253-5. There, it is stated that this technique may be unreliable for the identification of N.g. microorganism because the lectin agglutinates strains of other microorganisms such as Neisseria meningitidis (N.m.). Another problem with this technique is that autoagglutination of the sample may take place. This requires the use of fresh isolates to increase the percentage of identification of N.g.; otherwise, there could be a misdiagnosis in some percentage of N.g.

The aminopeptidase profiles of N.g. and N.m. have been studied and reported to be differential and capable of being reproducibly determined using chromogenic substrates, as set forth in Perrine, S., and Watson, R. R., Abstracts of the Annual Meeting of the American Society for Microbiology, p. 39 (1975). Thus, there is a disclosure that the presence of a reaction product of a substrate for gamma-glutamyl aminopeptidase (GGA), which does not react with N.g., is a positive indication that the specimen contains N.m. but not N.g. If this test is positive, it is an indication that the specimen contains N.m. but not N.g. However, if the test is negative, it is uncertain as to whether or not N.g. is present in the specimen. Another problem with this test is that the known substrates for

detecting gamma-glutamyl aminopeptidase activity require a long incubation for reaction, e.g. on the order of 2 hours at 37°C, thus resulting in a relatively slow test.

Hollis, D. G., et al, Appl. Microbiol., 17:71-77 (1969) described a lactose utilizing Neisseria species; and Corbett, P., and Ulivelli, A., J. Bacteriol., 95:52-57 (1968), described galactosidase activity of lactose-positive Neisseria. A substrate used to detect beta-galactosidase activity is ONPG (o-nitrophenyl-D-galactopyranoside [LeMinor, L., and Ben Hamida, F., Ann. Inst. Pasteur, 102:267 (1962)].

It is also known to detect leucine aminopeptidase with leucine-4-methoxy-beta-naphthylamide for a histochemical cell stain. Nachlas, M. M., et al, J. Biophys. Biochem. Cytol., 7:261 (1960).

It is a general object of the invention to provide a test for the differential detection of suspected N.g. and N.m. microorganisms which is both rapid and accurate.

The invention also enables in some embodiments the provision of an additional rapid test for the detection of B. catarrhalis.

It is a further aspect of the invention to provide a technique for reversing the mechanism of autoagglutination of microorganisms in the specimen, to reduce a false positive indication for agglutination tests.

Further features of the invention will be apparent from the following description of its preferred embodiments.

In general, a method has been provided for the differential detection of N.g. and N.m. microorganisms in a specimen. In a first step, a portion of the specimen is treated with lectin of a type which reacts with N-acetylglucosamine or N,N'-diacetylchitobiose to form an agglutinated product. Separate lectin tests preferably are used, including a WGA test for N.g. and a confirmatory lectin test for pathogenic Neisseria (using either soy bean agglutinin (SBA), potato Solanium Tuberosom agglutinin (STA) potato lectin, or both). For differentiating between N.g. and N.m., separate portions of the specimen are

treated with substrates specific for different enzymes in N.g. and N.m., and the reaction product is coupled with a diazo dye to form a visible color. Preferable fast substrates for N.m. and N.g. are gamma-glutamyl naphthylamide and proline naphthylamide, respectively, both substituted at the 4- and/or 6-positions with an electron donating group. This test can also be used in combination with the test for N.I.

microorganism, which is detected by contacting a portion of the specimen with a substrate which reacts with beta-galactosidase (BG) enzyme, and then detecting the presence of a reaction product formed by this enzyme, acting on the substrate, as a positive indication that the specimen contains N.l. A suitable substrate is ONPG. A further test may be performed for B. catarrhalis (B.c.) using a glycyl-prolyl naphthylamide also substituted at the 4- and/or 6-position.

In accordance with the present invention, a rapid accurate test is provided for various Neisseria, namely, N.g., N.m., N.l, and B.c. In a preferred embodiment a lectin agglutination test is used in combination with confirmation by rapid enzyme tests.

After growth of the microorganisms on selective culture media, for example Thayer-Martin culture media, a Gram Stain and oxidase test are performed to determine that the microorganisms are gram negative diplococci, giving a positive oxidase test. This is a presumptive indication that N.g., N.m., N.l. and/or B.c. may be present. A portion of the specimen is used for testing the presence of N.g. or N.m. microorganisms by agglutination with a lectin of a suitable type. For this purpose, one or more lectins are selected of a type which react with N-acetylglucosamine or N,N'-diacetylchitibiose. The presence of an agglutination reaction of the microorganism is a positive indication that the specimen contains either N.g. or N.m., or both.

Certain strains of the above Neisseria species tend to autoagglutinate. One portion of the specimen should be viewed to determine whether or not autoagglutination occurs in the absence of the lectin. If so, in accordance with the present invention, a procedure is provided for pretreating the specimen prior to lectin agglutination, for the purpose of reversing the mechanism of autoagglutination. In one such procedure, the specimen is treated with crude or purified DNA'ase, or a preparation containing DNA'ase, at an effective concentration

(e.g. 1 mg/ml). In another procedure, the specimen is treated with an agent which imparts a negative charge to the microorganisms in the specimen to cause individual microorganisms in an agglutinate to be repelled from each other. Suitable negative charge-imparting agents include succinic anhydride at an appropriate concentration (e.g. 0.25 mg/ml). These methods are of general applicability.

A lectin which has been suggested in the prior art to accomplish the objective of agglutination in the presence of N.g. is WGA. However, it has been found that not all N.g. microorganisms agglutinate in the presence of WGA. To solve this problem of false negative agglutination results, it is preferable to use different lectins in different tests. In suitable auxiliary lectin tests, a portion of the specimen is mixed with a lectin which causes agglutination of pathogenic Neisseria (N.m., N.g., N.l. and B.c.). Although this test is nonspecific, it serves to alert the technician that the WGA test produced a false negative. Suitable lectins for this auxiliary test are SBA or STA, alone or in combination.

For maximum accuracy, it has been found to be important to combine the above lectin with one or more confirmation tests to distinguish among the various pathogenic neisseria. In accordance with the present invention, one such confirmation test is derived from the knowledge that the N.m. contains GGA enzyme activity but not proline aminopeptidase (PA) activity. Conversely, N.g. contains PA activity but not GGA activity. Thus, a substrate which reacts with GGA, but not with PA, may be utilized as a positive indication that the specimen contains N.m. but not N.g. Specifically, such a substrate is added to a portion of the specimen, and the presence of a reaction product between the substrate and specimen is a positive indication that the specimen contains N.m. but not N.g. Amino acid beta-naphthylamides are known classes of substrates which release beta-naphthalamine chromogens as an indication of the presence of the enzyme. However, known substrates in this class such as gamma-glutamyl-beta-naphthylamide require an incubation on the order of 2 hours at 37°C for colorimetric detection through coupling with a diazo dye. Such substrates change colors on the addition of diazo dyes. In an alternative mode for detecting the substrate-enzyme reaction, fluorometric detection of the released naphthalamine may be used.

It has been found that the speed of the coupling reaction of the amino acid naphthylamide substrates is vastly increased by the substitution of an electron donating group R (e.g. an alkoxy group, such as methoxy, an hydroxy group, or a

halogen) at the 4-position, the 6-position, or both by reference to the following structure:

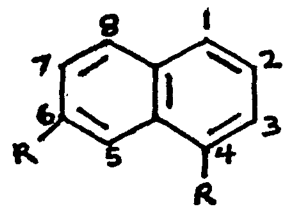

Thus, an effective substrate for the gamma-glutamyl aminopeptidase enzyme of N.m. is gamma-glutamyl-4-alkoxy-beta-naphthylamide, specifically with methoxy as the alkoxy group. Using this substrate and a suitable diazo coupler, the color changes in less than 10 minutes at an incubation temperature of 37°C for the same concentrations of reactants.

An analogous enzyme-substrate test to that for N.m. may be used for N.g. For this test, a substrate for the proline aminopeptidase enzyme portion of N.g. is added to a portion of the specimen. While unsubstituted proline-beta-naphthalamide may be used, a fast substrate comprises that class of compounds substituted at the 4-position, 6-position, or both, with an electron donating group of the foregoing type. A particularly effective substrate comprises proline-4-alkoxy-beta-naphthylamide, specifically the 4-methoxy compound.

Another analogous enzyme-substrate test may be used for B.c. For this test, a substrate for the glycyl-prolyl-aminopeptidase enzyme portion of N.g. is added to a portion of the specimen. A fast substrate comprises glycyl-prolyl-naphthylamide substituted at the 4-position, the 6-position, or both, with an electron donating group of the foregoing type. A particularly effective substrate is glycyl-prolyl-4-alkoxy-beta-naphthylamide, specifically the 4-methoxy compound.

Another feature of the invention is a test in combination with the above lectin agglutination and GGA enzyme test. In this test, the presence of N.l. microorganism in the specimen is detected by contacting a portion of the specimen with a substrate which reacts with BG enzyme (which is present on the N.l.). A reaction product formed between the BG enzyme on the N.l. microorganism is a positive indication that the specimen contains N.l. Suitable substrates for this purpose include ONPG, its bromo chloro-indoxyl-derivative and its beta-naphthyl-derivative. Specific substrates include o-nitrophenyl-D-galactopyranoside, p-nitrophenyl-D-galactopyranoside, phenolphthalein-D-galactopyranoside, 5-bromo-4-chloro-indoyl-D-galactopyranoside, bromothymolphthalein-D-galactopyranoside, and naphthyl-galactopyranoside.

By way of summary, four specific differential tests for the pathogenic neisseria (N.m., N.g., B.c. or N.l.) are set out in the following table:

| Substrate | N.m. | N.g. | B.c. | N.l. |
|---|---|---|---|---|
| ONPG | - | - | - | + |
| Proline-naphthylamide* | - | + | - | - |
| Gamma glutamyl-naphthylamide* | + | - | - | - |
| Gly-Pro-naphthylamide* | - | - | + | - |

* indicates substitution at the 4- or 6-position with an electron donating group. The "+" sign indicates a positive reaction with the specified neisseria, while the "-" sign indicates no reaction with the specified neisseria.

Suitable diazo coupling dyes for converting substrate-enzyme reaction products to colored form include the following: 4-amino-2,5-dimethoxy-4'-nitroazobenzene diazonium salt; tetrazotized o-dianisidine; diazotized-4'-amino-2',5'-diethoxybenzanilide zinc chloride salt; diazotized product of 4-benzoylamino-2,5-dimethoxyaniline zinc chloride; o-aminoazotoluene, diazonium salt (known as Fast Garnet); anthraquinone-1-diazonium chloride; 5-nitro-2-amino-methoxybenzene diazotate; N', N'-diethyl-4-methoxymethanilamide, diazonium salt; 2-amino-4-methoxybenzamide, diazonium salt; diazo-2-amino-5-chloroanisole, or diazo-5-chloro-o-anisidine; 5-chloro-2-toluidinediazonium chloride hemizinc chloride; 5-chloro-4-benzamido-2-methylbenzene diazonium chloride, hemizinc chloride; 6-benzamido-5-methoxy-m-toluidine diazonium chloride; and other diazonium salts.

An example of a suitable detection scheme in accordance with the present invention is as follows.

A specimen is grown on a Thayer-Martin culture plate. A Gram stain is performed on the microorganisms to ensure that a pure culture of Gram-negative diplococci is obtained from the Thayer-Martin plate. An oxidase test is performed to verify that the organisms are oxidase positive. A sufficient number of morphologically similar colonies are removed and emulsified in a suitable buffer such as phosphate buffered saline at a pH of 7.2 in a test tube.

The final turbidity of the bacterial suspension should be approximately equivalent to a McFarland No. 3 or No. 4, barium sulfate standard. In a test tube, or an agglutination plate or tray, a drop of WGA (approximately 1 mg/ml in aqueous solution or buffer solution) is added to one drop of the bacterial suspension. In another test tube, or agglutination plate or tray, a drop of phosphate buffered saline is added to another drop of bacterial suspension. In a third test tube or agglutination plate/tray, a drop of SBA or STA (approximately 1 mg/ml in aqueous solution of buffer solution) is added to a drop of bacterial suspension. [All test tubes/plates/trays are shaken.] The saline control should show a negative agglutination after being shaken. If the saline control is positive, this indicates that there is autoagglutination and the specimen should be treated, and the autoagglutination tests with WGA and SBA/STA should be performed with treated bacterial suspensions. Suitable treatment for reversing autoagglutination provides the addition of .005 ml succinic anhydride solution to 0.2 ml of the specimen, prior to performance of the agglutination tests with WGA and SBA. A positive agglutination reaction with WGA and SBA/STA indicates the presence of N.g. or non-encapsulated M.c. A negative agglutination with WGA, but positive agglutination with SBA/STA indicates the possibility of N.m. or N.g. whose surface structure has been altered.

One drop of the specimen is then added separately to one drop of each of the following substrate solutions; namely, gamma-glutamyl-4-methoxy-beta-naphthylamide, prolyl-4-methoxy-beta-naphthylamide, ONPG, and glycyl-prolyl-4-methoxy-beta-naphthylamide, each at a concentration of 1 mg/ml in Tris-NCl buffer or phosphate buffer. A positive test for ONPG, indicated by a color change from colorless to yellow within 10 minutes at 37°C, indicates the presence of N.l. A positive test for GGA, indicated by a color change to red or pink in the gamma-glutamyl-4-methoxy-beta-naphthylamide containing solution after 10 minutes of incubation at 37°C with the bacterial suspension, and after the addition of Fast Garnet GBC salt (at a concentration of approximately 0.2 mg/ml in aqueous solution), indicates the presence of N.m. A positive test for PA, indicated by a color change to red or pink in the prolyl-4-methoxy-beta-naphthylamide containing solution after 10 minutes of incubation at 37°C with the bacterial suspension, and after the addition of Fast Garnet GBC salt solution, indicates the presence of N.g. A positive test for GPA, indicated by a color change to red or pink in the glycyl-prolyl-4-methoxy-beta-naphthylamide containing solution after 20 minutes of incubation at 37°C with the bacterial suspension, and after the addition of Fast Garnet GBC solution, indicates the presence of B.c.

The above tests can be performed on a single plate including wells and simultaneously, to provide a rapid identification of N.g., N.m., and N.l. (i.e., in less than 30 minutes). Furthermore, the accuracy of identification is increased by the use of SBA or STA in combination with WGA, and by the use of treatment of the specimen with DNA'ase and/or succinic anhydride where autoagglutination is a problem.

CLAIMS:

1. A method for the differential detection of suspected Neisseria gonorrhoeae and Neisseria meningitidis microorganisms in a specimen, comprising:

(a) contacting a portion of the specimen containing suspected Neisseria gonorrhoeae and/or Neisseria meningitidis microorganisms with lectin of a type which reacts with N-acetylglucosamine or N,N'-diacetyl-chitobiose, and detecting the presence of an agglutina-reaction formed between the lectin and N-acetylglucosamine or N,N'-diacetylchiobiose on the surface of the Neisseria gonorrhoeae or Neisseria meningitidis microorganism as a positive indication that the specimen contains either Neisseris gonorrhoeae, Neisseria meningitidis, or both; and

(b) contacting a portion of the specimen containing suspected Neisseria gonorrhoeae and/or Neisseria meningitidis microorganisms with a substrate specific for gamma-glutamyl aminopeptidase which reacts with Neisseria meningitidis or a substrate specific for proline aminopeptidase which reacts with Neisseria gonorrhoeae, and detecting the presence of a reaction product formed between said substrate and specimen portion as a positive indication that the specimen contains Neisseria menigitids or Neisseria gonorrhoeae.

2. A method as claimed in claim 1 in which the lectin comprises wheat germ agglutinin.

3. A method as claimed in claim 1 or claim 2 also including (c) contacting a portion of the specimen with a lectin comprising soy bean agglutinin and/or with a lectin comprising STA potato lectin.

4. A method as claimed in any one of claims 1 to 3 in which the reaction of (a) forms an agglutinate which is visibly detectable.

5. A method as claimed in any one of claims 1 to 4

in which the substrate in (b) comprises gamma-glutamyl naphthylamide substituted at the 4-position, the 6-position, or both, with an electron donating group, e.g. gamma glutamyl-4-alkoxy-beta-naphthylamide.

6. A method as claimed in any one of claims 1 to 4 in which the substrate in (b) comprises proline naphthylamide substituted at the 4-position, the 6-position, or both, with an electron donating group, e.g. proline-4-alkoxy-beta-naphthylamide.

7. A method as claimed in any one of claims 1 to 6 also including (c') contacting a portion of the specimen with a substrate specific for glycyl-prolyl-aminopeptidase which reacts with B. catarrhalis, and detecting the presence of a reaction product formed between the substrate and specimen portion as a positive indication that the specimen contains B. catarrhalis.

8. A method as claimed in claim 7 in which the substrate in (C') comprises glycyl-prolyl-naphthylamide substituted at the 4-position, the 6-position, or both, with an electron donating group, e.g. glycyl-prolyl-4-alkoxy-beta-naphthylamide.

9. A method as claimed in any one of claims 5, 6 or 8 in which the reaction product of (b) and/or (c') is coupled with a diazo dye developer to form a visible color.

10. A method as claimed in any one of claims 1 to 9 in which the presence of Neisseria lactamica microorganism in the specimen is detected by the following:

(c") contacting a portion of the specimen with a substrate which reacts with beta-galactosidase enzyme, and detecting the presence of a reaction product formed between beta-galactosidase enzyme as a positive indication that the specimen contains Neisseria lactomica.

11. A method as claimed in claim 10 in which the substrate for beta-galactosidase enzyme is o-nitrophenyl-D-galactopyranoside, p-nitrophenyl-D-galactopyranoside, phenophthalein-D-galactopyranoside, 5-bromo-4-chloro-indoyl-D-galactopyranoside, bromothylmolphthalein-D-galactopyranoside, or naphthyl-galactopyranoside.

12. A method as claimed in any one of claims 1 to 11 in which prior to (a), the specimen is treated with an agent which reverses the mechanism of autoagglutination of microorganisms in the specimen.

13. A method as claimed in claim 12 in which the specimen is treated with DNA'ase or with an agent which imparts a negative charge to the microorganisms to cause individual microorganisms in an agglutinate to be repelled from each other, e.g. a negative charge-imparting agent comprising a closed ring anhydride.

14. A method for separating DNA-based microorganisms in an agglutinated state into individual microorganisms comprising the step of treating the agglutinate with DNA'ase or a negative charge-imparting agent, e.g. a closed ring anhydride.

15. A fast method for detecting the enzyme portion of a Neisseria which is Neisseria gonorrhoeae, Neisseria meningitidis or B. catarrhalis, comprising:

(a) contacting the Neisseria with a fast substrate which is gamma-glutamyl naphthylamide, proline naphthylamide or glycyl-prolyl-naphthylamide, each substrate being substituted at the 4-position, the 6-position, or both with an electron donating group, and

(b) coupling the reaction product of (a) with a diazo dye developer to form a visible color.

16. A kit adapted for use in the performance of a method as claimed in any one of claims 1 to 13 or in a method as claimed in claim 14 or in a method as claimed in claim 15, and comprising a container containing a lectin of a type which reacts with N-acetylglucosamine or N,N'-diacetylchitobiose, or a substrate specific for gamma-glutamyl aminopeptidase which reacts with Neisseria meningitidis , e.g. gamma-glutamyl naphthylamide, or a substrate specific for proline aminopeptidase which reacts with Neisseria gonorrhoeae, e.g. proline naphthylamide, or a substrate specific for glycyl-prolyl-aminopeptidase which reacts with B. catarrhalis, e.g. glycyl-prolyl-naphthylamide, each of the aforesaid specifically named substrates being substituted at the 4-position, the 6-position, or both, with an electron donating group, or a substrate which reacts with beta-galactosidase enzyme, or DNA'ase, or a negative charge-imparting agent, said container being associated with instructions for the performance of one or more methods as aforesaid and as appropriate.

European Patent Office

# EUROPEAN SEARCH REPORT

0122023

EP 84 30 1507

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| Y | WO-A-8 002 433 (NATIONAL RESEARCH DEVELOPMENT CORPORATION)<br>* Pages 2,3,5; page 7, lines 8-12; pages 11,13,22,23 * | 1,5,6, 9,10, 11,15, 16 | C 12 Q 1/12<br>C 12 Q 1/36<br>C 12 Q 1/34 |
| Y | GB-A-2 031 949 (AMERICAN HOME PRODUCTS CORP.)<br>* Whole document * | 1,5,9-11,15, 16 | |
| D,Y | US-A-4 298 689 (R.J. DOYLE et al.)<br>* Whole document * | 1,10, 16 | |
| A | | 2,4 | TECHNICAL FIELDS SEARCHED (Int. Cl. 3) |
| A | US-A-3 862 011 (R.E. SMITH)<br>* Whole document * | 1,5,9, 15,16 | C 12 Q<br>G 01 N |
| A | FR-A-2 286 192 (WARNER-LAMBERT COMPANY)<br>* Whole document * | 10,11 | |
| | --- -/- | | |

The present search report has been drawn up for all claims

| Place of search | Date of completion of the search | Examiner |
|---|---|---|
| THE HAGUE | 25-06-1984 | GRIFFITH G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82

European Patent Office

# EUROPEAN SEARCH REPORT

0122023

EP 84 30 1507



## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| A | CHEMICAL ABSTRACTS, vol. 54, 1960, column 21294c,d,e, Columbus, Ohio, US M.M. NACHLAS et al.: "Improvement in the histochemical localization of leucine aminopeptidase with a new substrate, L-leucyl-4-methoxy-2-naphthamide" & J. BIOPHYS. BIOCHEM. CYTOL. 7, 261-4(1960)<br>----- | | |
| | | | TECHNICAL FIELDS SEARCHED (Int. Cl. 3) |

The present search report has been drawn up for all claims

| Place of search | Date of completion of the search | Examiner |
|---|---|---|
| THE HAGUE | 25-06-1984 | GRIFFITH G. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82